# EUROPÄISCHE PATENTANMELDUNG

(11) **EP 1 114 624 A1**
(43) Veröffentlichungstag der Anmeldung: **11.07.2001**
(21) Anmeldenummer: 00811140.3
(22) Anmeldetag: 01.12.2000
(51) Int. Cl.: A61F 2/34, A61F 2/36

(54) **Stossabsorbierende Gelenkpfanne**

(30) Priorität: 04.01.2000 EP 00810001
(71) Anmelder: Sulzer Orthopedics Ltd., 6340 Baar (CH)
(72) Erfinder: Spotorno, Lorenzo, Prof. Dr. med., 17024 Finale Ligure (IT); Bolon, Edouard, 8405 Winterthur (CH)
(74) Vertreter: Sulzer Management AG

(57) **Zusammenfassung**

Eine künstliche Gelenkpfanne (1) umfasst eine Aussenschale (2) zur Befestigung im Knochen und einen in dieser Aussenschale (2) angeordneten Einsatz (3) zur Aufnahme eines Gelenkkopfs. Die Aussenschale (2) weist ein bewegliches Segment (20) auf, das durch in Meridianen verlaufende Schlitze begrenzt ist. Der Einsatz (3) ist so ausgebildet, dass zwischen der Innenfläche der Aussenschale (2) und der Aussenfläche des Einsatzes (3) ein lichter Raum (23) gebildet wird. Das bewegliche Segment (20) der Aussenschale (2) ist zusätzlich zu den in Meridianen verlaufenden Schlitzen auch noch durch in äquatorialer Richtung verlaufende Schlitze (22) begrenzt.

## Beschreibung

Die Erfindung betrifft eine künstliche Gelenkpfanne gemäss dem unabhängigen Patentanspruch.

Künstliche Gelenkpfannen gibt es in zahlreichen Ausführungen. Die Tendenz geht dahin, immer dann, wenn dies möglich ist, die künstliche Gelenkpfanne zementfrei zu implantieren. Bei der Implantation einer künstlichen Gelenkpfanne wird der Beckenknochen des Patienten vorbereitet, indem er beispielsweise mit einem kugelkalottenförmigen Fräser ausgefräst wird, sodass in die so erstellte Kavität hinein die künstliche Gelenkpfanne eingebracht werden kann. Bei den zementfrei zu implantierenden künstlichen Gelenkpfannen wird die künstliche Gelenkpfanne dabei auf verschiedene Art und Weise am Knochen befestigt. So kann z.B. die Aussenschale mittels Knochenschrauben befestigt werden. Andere Varianten sehen vor, dass die Aussenschale ein Gewinde oder Gewindeteile auf ihrer Aussenfläche aufweist und dieses Gewinde bzw. diese Gewindeteile und damit die Aussenschale selbst direkt in den Knochen eingeschraubt wird. Wieder eine andere Variante sieht vor, dass Strukturen auf der Aussenfläche der Aussenschale vorgesehen sind, die beim Einschlagen der künstlichen Gelenkpfanne in die ausgefräste Kavität hineingleiten und sich dann in den Knochen eingraben bzw. im Knochen verhaken.

Die Befestigung der Aussenschale der künstlichen Gelenkpfanne am Knochen ist deshalb wichtig, weil die Primärstabilität der künstlichen Gelenkpfanne - also die Stabilität der künstlichen Gelenkpfanne in der Phase unmittelbar nach der Operation und in der Zeit, bis der Knochen auf die Aussenfläche der Gelenkpfanne aufgewachsen ist - sichergestellt sein muss, damit der Patient so schnell wie möglich nach der Operation seine Mobilität wiedergewinnt.

In der Aussenschale einer solchen künstlichen Gelenkpfanne ist eine Innenschale angeordnet, welche eine sphärische Artikulationsfläche aufweist, in welcher nach der Implantation der künstlichen Gelenkpfanne und des zugehörigen Femurschafts die Gelenkkugel des Femurschafts angeordnet ist. Dabei dürfen sich selbst unter Belastung weder die Artikulationsfläche der Innenschale der künstlichen Gelenkpfanne noch die Gelenkkugel merkbar verformen, weil sonst die Beweglichkeit der Gelenkkugel nicht mehr gewährleistet ist. Dies gilt umso mehr für Metall-Metall Gleitpaarungen, also für Gleitpaarungen, bei denen sowohl die Gelenkkugel als auch die Artikulationsfläche der Innenschale (bzw. oft sogar die gesamte Innenschale) metallisch ist. Solche Metall-Metall Gleitpaarungen sind praktisch verschleissfrei bzw. ausgesprochen verschleissarm und auch formstabil, dürfen aber auch nur sehr geringe Formabweichungen und sehr geringe Fertigungstoleranzen aufweisen.

Bei Belastungen kann der die Gelenkpfanne umgebende Beckenknochen sich elastisch verformen, sodass bei örtlichen Belastungen und speziell bei Stossbelastungen einzelne Bereiche der künstlichen Gelenkpfanne sich zeitweise von der Aussenschale abheben können und damit andere Bereiche des Knochens relativ hoch beansprucht werden können, weil die gesamte auftretende Belastung nur auf diejenigen Bereiche des Knochens übertragen werden kann, die in Kontakt mit der Aussenschale der künstlichen Gelenkpfanne stehen.

Um dies zu verhindern oder zu mildern, ist bereits in der EP-A-0,640,325 eine künstliche Gelenkpfanne vorgeschlagen worden, welche mehrere elastische Segmente aufweist, die durch ihre Elastizität derartige elastische Verformungen des Knochens mitmachen können, wodurch sie in Kontakt mit dem Knochen bleiben und die Belastung auch über diese Segmente auf den Knochen übertragen werden kann. Die Segmente sind durch in Medianen hin zum Pol verlaufende Schlitze begrenzt. Zwischen der Aussenschale und der Innenschale ist eine Zwischenschale angeordnet, deren Aussenfläche in einem Stützbereich an der Innenfläche der Aussenschale anliegt, wobei im übrigen zwischen der Aussenfläche der Zwischenschale und der Innenfläche der Aussenschale einen lichter Raum begrenzt wird. Dieser Raum steht insbesondere für eine elastische Verformung der Aussenschale zur Verfügung.

Bei der Implantation der in der EP-A-0,640,325 vorgeschlagenen künstlichen Gelenkpfanne wird die Aussenschale unter Vorspannung in die vorbereitete Kavität im Knochen eingebracht, da sie nach dem Einbringen ja die notwendige Primärstabilität gewährleisten muss, ohne dass Knochenzement beteiligt ist. Die Kavität weist also gegenüber den Abmessungen der Aussenschale der künstlichen Gelenkpfanne im entspannten Zustand ein Untermass auf. Dazu wird bei der Präparation der Kavität typischerweise ein Kugelfräser mit einem Aussendurchmesser gewählt, der geringer ist als der Aussendurchmesser der Aussenschale im entspannten Zustand.

Diese in der EP-A-0,640,325 vorgeschlagene künstliche Gelenkpfanne stellt für sehr viele Fälle eine gut funktionierende Lösung dar, weist aber noch gewisse Verbesserungsmöglichkeiten auf. So sind die einzelnen Segmente der Aussenschale der in der EP-A-0,640,325 vorgeschlagenen künstlichen Gelenkpfanne mehr oder weniger nur in radialer Richtung elastisch verformbar, weil ja nur in Medianen verlaufende Schlitze vorgesehen sind. Die elastische Verformung des Knochens hingegen erfolgt nicht ausschliesslich in radialer Richtung, sondern hängt von der Art der auftretenden Belastung ab. In Umfangsrichtung sind die einzelnen Segmente der Aussenschale jedoch praktisch nicht elastisch verformbar, weil sie in Umfangsrichtung betrachtet doch eine gewisse Breite aufweisen müssen, damit eine genügend grosse Kontaktfläche zum Knochen existiert.

Darüberhinaus hält sich die elastische Verformbarkeit der einzelnen Segmente auch in radialer Richtung innerhalb gewisser Grenzen. Dies gilt umso mehr, als die Aussenschale praktisch überall eine mehr oder weniger konstante Dicke aufweist bzw. allenfalls im äquatorialen Bereich noch etwas dicker ausgebildet ist als im Polbereich. Gerade im äquatorialen Bereich ist aber die elastische Verformbarkeit der Aussenschale insofern wichtig als dort die Primärverankerung der künstlichen Gelenkpfanne hauptsächlich erfolgt, und ein Abheben des Knochens von der Aussenschale dort sogar eine Verschlechterung der Primärverankerung zur Folge haben kann.

Es ist daher eine Aufgabe der Erfindung, eine entsprechend verbesserte künstliche Gelenkpfanne vorzuschlagen, deren Aussenschale eine Verformbarkeit aufweist, die der elastischen Verformung des Knochens auch in Umfangsrichtung folgen kann, um örtliche Belastungen und speziell Stossbelastungen möglichst über eine grosse Kontaktfläche auf den Knochen übertragen zu können. Darüberhinaus soll die Gelenkpfanne eine möglichst gute Primärverankerung gerade im äquatorialen Bereich aufweisen.

Diese Anforderungen werden durch eine erfindungsgemässe künstliche Gelenkpfanne, wie sie durch die Merkmale des unabhängigen Patentanspruchs charakterisiert ist, gelöst. Besonders vorteilhafte Ausgestaltungen der erfindungsgemässen künstlichen Gelenkpfanne ergeben sich aus den Merkmalen der abhängigen Patentansprüche.

Insbesondere ist bei der erfindungsgemässen künstlichen Gelenkpfanne das bewegliche Segment (bzw. im Falle von mehreren beweglichen Segmenten sind die beweglichen Segmente) der Aussenschale zusätzlich zu den in Meridianen verlaufenden Schlitzen auch noch durch in äquatorialer Richtung verlaufende Schlitze begrenzt. Dies hat zur Folge, dass die beweglichen Segmente zusätzlich zu ihrer elastischen Verformbarkeit in radialer Richtung auch noch in Umfangsrichtung elastisch Verformbar sind. Dadurch wird erreicht, dass die Segmente bei einer elastischen Verformung des Knochens sich ebenfalls elastisch verformen können und damit eine sehr grosse Kontaktfläche zum Knochen erhalten bleiben kann, auch bei örtlich auftretenden Belastungen und speziell auch bei Stossbelastungen.

Die Schlitze verlaufen also so, dass das Segment bzw. die Segmente sowohl in radialer Richtung als auch in äquatorialer Richtung beweglich ist. Dies ist auch insofern von Vorteil, als es aufgrund des häufig doch recht beengten Operationsfelds auch einmal zu nicht ganz gleichmässig vorbereiteten (ausgefrästen) Kavitäten kommen kann. Solche Ungleichmässigkeiten können dann durch die elastische Verformbarkeit der Segmente ausgeglichen werden. Ohnehin wird die Gelenkpfanne ja unter einer Vorspannung implantiert, d.h. die Kavität weist gegenüber den Abmessungen der künstlichen Gelenkpfanne im entspannten Zustand ein Untermass auf.

Bei einem vorteilhaften Ausführungsbeispiel der erfindungsgemässen künstlichen Gelenkpfanne verlaufen die Schlitze, welche das bewegliche Segement der Aussenschale begrenzen, so, dass die Aussenschale mindestens ein T-förmiges Segment aufweist, welches seinen Fuss im Polbereich hat und sein Dach im äquatorialen Bereich. Diese T-förmige Form des Segments bzw. der Segmente zeichnet sich vor allem dadurch aus, dass speziell im äquatorialen Bereich der Aussenschale stets eine grosse Kontaktfläche zum Knochen gegeben ist, auch bei elastischen Verformungen des Knochens in Umfangsrichtung, gleichzeitig jedoch auch eine gute elastische Verformbarkeit der Gelenkpfanne in radialer Richtung gegeben ist. Dies ist insofern wichtig, weil die Primärverankerung der Gelenkpfanne im wesentlichen im äquatorialen Bereich erfolgt und somit stets eine gute Verankerung der Gelenkpfanne im äquatorialen Bereich gegeben ist. Grundsätzlich kommen aber auch andere geometrische Ausgestaltungen der Segmente wie z.B. L-förmige Segmente in Betracht.

Besonders vorteilhaft erweist sich dabei eine künstliche Gelenkpfanne, bei welcher die Aussenschale drei T-förmige Segmente aufweist, weil dabei die einzelnen Dächer der T-förmigen Segmente in Umfangsrichtung betrachtet eine genügend grosse Breite aufweisen, um eine gute elastische Verformbarkeit in Umfangsrichtung aufzuweisen. Grundsätzlich sind aber auch andere Zahlen solcher T-förmiger Segmente vorstellbar von einem bis hin zu sechs Segmenten oder mehr. Drei Segmente sind jedoch besonders vorteilhaft.

Bei einem weiteren vorteilhaften Ausführungsbeispiel der künstlichen Gelenkpfanne nimmt die Dicke der Aussenschale in Richtung vom Polbereich zum äquatorialen Bereich hin ab. Diese Abnahme der Dicke hat zur Folge, dass die elastisch verformbaren Segmente in Richtung zum äquatorialen Bereich hin betrachtet immer weichere "Biegefedern" darstellen. Dadurch wird im äquatorialen Bereich auch bei elastischen Verformungen des Knochens stets eine grosse Kontakfläche der Aussenschale zum Knochen gewährleistet. Dies ist einerseits für eine gute Primärverankerung von Vorteil, die ja hauptsächlich im äquatorialen Bereich erfolgt, andererseits ermöglicht dies auch, dass sich die Biegefedern bei einer stossartigen Belastung besser verformen können und so die stossartige Belastung besser abfedern können.

Bei einem weiteren vorteilhaften Ausführungsbeispiel der erfindungsgemässen künstlichen Gelenkpfanne ist die Aussenschale im äquatorialen Bereich über die Fortsetzung der Kontur des Aussenschale nach aussen hin vorstehend ausgebildet. In diesem Bereich der Aussenschale sind auf der Aussenwand Mittel zur Befestigung der Aussenschale im Knochen vorgesehen. Diese Ausführungsform erlaubt eine besonders gute Verankerung der Aussenschale im Knochen. Dazu stelle man sich vor, dass im Knochen bereits eine sphärische Kavität mit Hilfe eines Kugelfräsers vorbereitet worden ist. Steht nun im äquatorialen Bereich die Aussenschale über die Fortsetzung der Kontur der Aussenschale nach aussen hin vor, so müssen die beweglichen Segmente elastisch verformt werden, damit die Aussenschale in die vorbereitete Kavität hinein eingebracht werden kann. Dazu müssen die elastisch verformbaren Segmente bis auf ein Mass verformt werden, welches z.B. der Fortsetzung der Kontur der Aussenschale entspricht, wodurch die Aussenschale in die vorbereitete Kavität im Knochen hineingleiten kann (oder sie wird in die Kavität eingeschlagen, wodurch die Deformation der einzelnen Segmente erfolgt). Durch die zusätzlichen Mittel auf der Aussenschale in diesem Bereich, beispielsweise eine Verzahnung, kann ein zusätzliches Verhaken der Aussenschale in diesem Bereich und damit eine noch bessere Primärstabilität bewirkt werden. Darüberhinaus bewirkt eine solche Verzahnung bei wechselnden Belastungen, dass das Knochenwachstum angeregt wird und somit das Einwachsen der Aussenschale in den Knochen bzw. das Aufwachsen des Knochens auf die Aussenschale begünstigt wird.

Ein weiteres vorteilhaftes Ausführungsbeispiel der erfindungsgemässen künstlichen Gelenkpfanne zeichnet sich dadurch aus, dass der Einsatz im Bereich des Pols der Aussenschale fest mit der Aussenschale verbunden ist. Die Hauptbelastungsrichtung weicht aber typischerweise von der Polachse ab, sodass die Belastung (z.B. ein Stoss) regelmässig über die Verbindung des Einsatzes am Pol in die Aussenschale eingeleitet wird und von dort gegebenenfalls durch eine elastischen Verformung der Aussenschale relativ zum Einsatz abgefedert an den Knochen weitergegeben wird.

Besonders vorteilhaft ist in diesem Zusammenhang ein Ausführungsbeispiel einer künstlichen Gelenkpfanne, bei welcher die Verbindung des Einsatzes mit der Aussenschale durch einen zylindrischen Zapfen gebildet wird, welcher in eine entsprechende Öffnung der Aussenschale eingepresst ist. Dieses Ausführungsbeispiel kann herstellerseitig vormontiert ausgeliefert werden, ist einfach in der Herstellung der einzelnen Komponenten, und der Einsatz ist zuverlässig und unlösbar mit der Aussenschale verbunden.

Wie eingangs bereits erwähnt, sind solche künstlichen Gelenkpfanne besonders bevorzugt, bei welcher sowohl die Aussenschale als auch der Einsatz metallisch sind, wobei die Aussenschale vorzugsweise aus Titan bzw. aus einer Titanlegierung und der Einsatz vorzugsweise aus einer Kobalt-Chrom-Legierung hergestellt ist. Bei diesem Ausführungsbeispiel handelt es sich typischerweise um eine Metall-Metall Gleitpaarung, weil die Gelenkkugel der anderen Gelenkkomponente dann ebenfalls regelmässig metallisch ist. Der Einsatz selbst ist bei diesem Ausführungsbeispiel starr, ebenso wie die Gelenkkugel, was aufgrund der hohen erforderlichen Formgenauigkeit und der geringen zulässigen Fertigungstoleranzen bei Metall-Metall Gleitpaarungen auch erforderlich ist. Die Vorteile der Elastizität der künstlichen Gelenkpfanne werden durch die Ausbildung der Aussenschale erreicht.

Weitere vorteilhafte Ausgestaltungen ergeben sich aus der nachfolgenden Beschreibung von Ausführungsbeispielen anhand der Zeichnung. Es zeigen, teilweise in schematischer Darstellung und/oder im Schnitt:
- Fig. 1: ein erstes Ausführungsbeispiel einer erfindungsgemässen künstlichen Gelenkpfanne in perspektivischer Darstellung,
- Fig. 2: eine Ansicht des Ausführungsbeispiels gemäss Fig. 1 in einer Ansicht von unten,
- Fig. 3: einen Schnitt durch das Ausführungsbeispiel gemäss der Linie III-III in Fig. 2
und
- Fig. 4: einen ähnlichen Schnitt wie in Fig. 3 durch ein zweites Ausführungsbeispiel einer erfindungsgemässen künstlichen Gelenkpfanne.

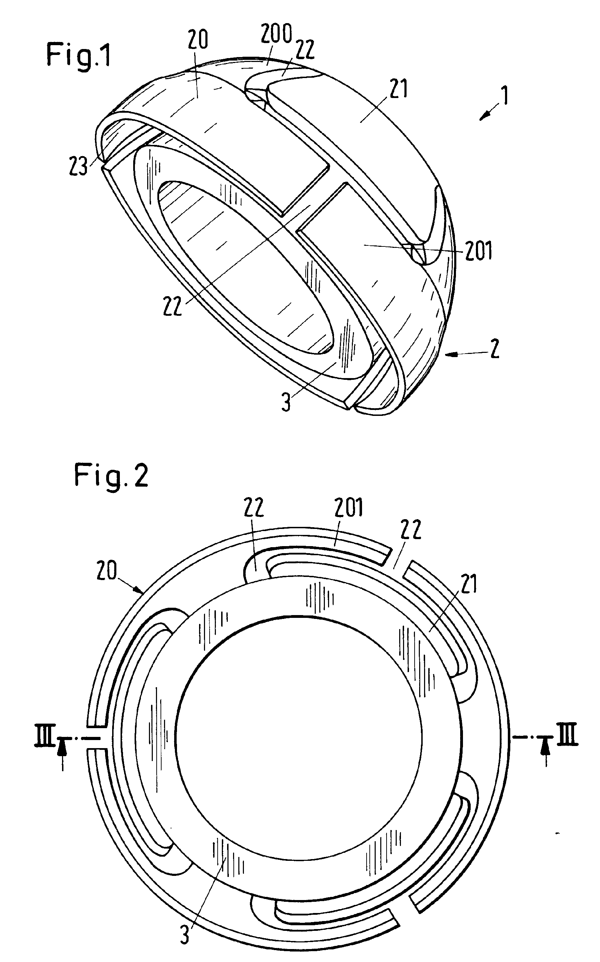

In Fig. 1-3 erkennt man ein erstes Ausführungsbeispiel einer erfindungsgemässen Gelenkpfanne 1, welche eine Aussenschale 2 zur Befestigung am Knochen und einen in dieser Aussenschale 2 angeordneten Einsatz 3 aufweist. Die Aussenschale 2 weist mehrere bewegliche Segmente 20 bzw. 21 auf, wobei sich die Beweglichkeit der Segmente 21 in gewissen Grenzen hält. Zwischen der Aussenschale 2 und dem Einsatz 3 wird ein lichter Raum 23 gebildet.

Die Segmente 20 und 21 sind durch Schlitze 22 begrenzt, welche sowohl in Meridianen verlaufen (also in Richtung Äquator/Pol) als auch in äquatorialer Richtung. Bei dem gezeigten Ausführungsbeispiel sind drei Segmente 20 vorgesehen (und entsprechend drei Segmente 21), grundsätzlich kann aber die Anzahl beweglicher Segement im Bereich von eins bis sechs oder sogar mehr liegen. Die Segmente 20 weisen bei dem gezeigten Ausführungsbeispiel eine T-förmige Gestalt auf, wobei das T-förmige Segment 20 seinen Fuss 200 im Polbereich hat und sein Dach 201 im äquatorialen Bereich. Die T-förmigen Segmente 20 sind in radialer Richtung beweglich, also nach innen bzw. nach aussen, weil der Fuss 200 der T-förmigen Segmente 20 als "Biegefeder" in dieser Richtung wirkt. Sie sind aber auch in Umfangsrichtung beweglich, weil das Dach 201 der T-förmigen Segmente als "Biegefeder" in dieser Richtung wirkt. Die Segmente 20 fungieren folglich als jeweils zwei "Biegefedern", nämlich sowohl als "Biegefeder" in radialer Richtung als auch als "Biegefeder" in Umfangsrichtung.

Der Einsatz 3 ist bei dem gezeigten ersten Ausführungsbeispiel der erfindungsgemässen künstlichen Gelenkpfanne 1 im Bereich des Pols der künstlichen Gelenkpfanne 1 fest mit der Aussenschale 2 verbunden. Diese Verbindung des Einsatzes 3 mit der Aussenschale 2 wird durch einen zylindrischen Zapfen 34 an dem Einsatz 3 gebildet, welcher hier in eine entsprechende Öffnung 24 der Aussenschale 2 eingepresst ist. Dadurch kann die erfindungsgemässe Gelenkpfanne herstellungsseitig fertig montiert und steril verpackt ausgeliefert werden.

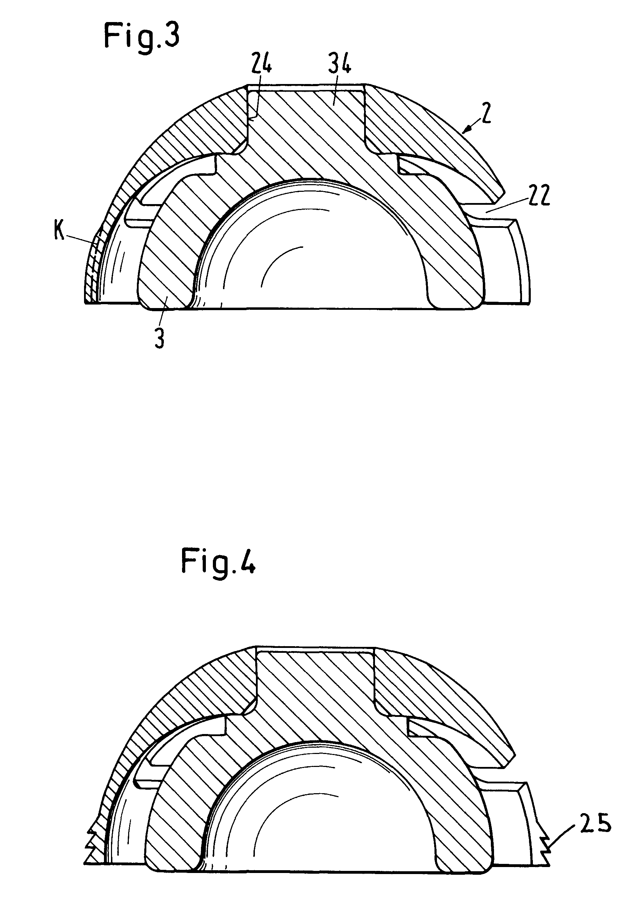

Wie man insbesondere in Fig. 3 erkennen kann, nimmt die Dicke der Aussenschale 2 in Richtung vom Polbereich hin zum äquatorialen Bereich der künstlichen Gelenkpfanne ab. Dies hat zur Folge, dass die Federhärte der als "Biegefedern" fungierenden T-förmigen Segmente 20 zum äquatorialen Bereich hin abnimmt. Ferner erkennt man in Fig. 3, dass die Aussenschale 2 im äquatorialen Bereich über die Fortsetzung der Kontur der Aussenschale 2 nach aussen hin vorstehend ausgebildet ist, wobei die Fortsetzung der Kontur der Aussenschale 2 durch die gestrichelte Linie K angedeutet ist.
Bei der Implantation der künstlichen Gelenkpfanne wird zunächst im Beckenknochen des Patienten eine Kavität hergestellt, typischerweise mit Hilfe eines Kugelfräsers, dessen Aussendurchmesser beispielsweise in etwa dem Aussendurchmesser der Aussenschale 2 der künstlichen Gelenkpfanne 1 entspricht oder geringfügig geringer ist. In jedem Fall ist der Aussendurchmesser des Kugelfräsers und somit auch der Durchmesser der Kavität geringer als der Aussendurchmesser der Aussenschale 2 im äquatorialen Bereich, wo ja die Aussenschale 2 über die Fortsetzung K der Kontur der Aussenschale nach aussen vorsteht.

Beim Einbringen der künstlichen Gelenkpfanne 1 in die präparierte Kavität werden also die T-förmigen Segmente 20 unter Vorspannung in die Kavität eingebracht. Das ist auch wichtig, weil die Primärverankerung ja zementfrei erfolgt und bei diesem Ausführungsbeispiel auch keinerlei sonstige Befestigungsmittel für die Aussenschale 2 am Knochen vorgesehen sind, wie z.B. Knochenschrauben. Somit sorgen die unter Vorspannung in die präparierte Kavität eingebrachten T-förmigen Segmente für eine gute Primärverankerung der künstlichen Gelenkpfanne 1 im Knochen.

Bei dem zweiten Ausführungsbeispiel der erfindungsgemässen Gelenkpfanne gemäss Fig. 4 sind in dem äquatorialen Bereich, in welchem die Aussenschale 2 über die Fortsetzung der Kontur K der Aussenschale 2 nach aussen vorstehend ausgebildet ist, zusätzliche Mittel zur Befestigung der Aussenschale im Knochen vorgesehen. Diese Mittel sind in Fig. 4 in Form von Finnen 25 dargestellt, können aber beispielsweise auch als Zähne ausgebildet sein. Diese Finnen 25 wirken als zusätzliche Verbesserung der Primärverankerung der Aussenschale 2 im Knochen, sie regen aber auch bei wechselnden Belastungen das Knochenwachstum an, wodurch der Knochen besser auf die Aussenschale 2 aufwachsen kann, was die Sekundärstabilität fördert.

Bei den gezeigten Ausführungsbeispielen ist sowohl der Einsatz 3 als auch die Aussenschale 2 metallisch. Der Einsatz 3 ist vorzugsweise aus einer Kobalt-Chrom Legierung hergestellt, während die Aussenschale aus Titan oder einer Titanlegierung hergestellt ist, die ggf. auf der Aussenfläche porös ausgebildet sein kann, um ein besseres Einwachsen in den Knochen zu fördern.

Der Einsatz 3 ist starr ausgebildet, weil vorzugsweise die Gelenkkugel (nicht dargestellt) ebenfalls aus Metall hergestellt ist. Da in diesem Fall die Formgenauigkeit sehr hoch sein muss und die Fertigungstoleranzen nur sehr gering sein dürfen, darf sich der Einsatz 3 unter Belastung auch nicht verformen. Das wiederum heisst bei den gezeigten Ausführungsbeispielen, dass z.B. bei einem Stoss, der typischerweise nicht in Richtung der Polachse erfolgt (die Hauptbelastungsrichtung weicht ja regelmässig von der Richtung der Polachse ab, weil die künstliche Pfanne entsprechend der natürlichen Gelenkpfanne implantiert wird) die Belastung über den eingepressten Zapfen 34 des Einsatzes 3 auf die Aussenschale 2 übertragen wird, die aufgrund ihrer "federnden" Eigenschaften den Stoss dämpfen kann, dabei aber stets mehr oder weniger in vollem Kontakt mit dem Knochen bleibt, wodurch die lokal auftretenden Belastungen sich in unkritischen Grenzen halten.

Was die Herstellung einer solchen künstlichen Gelenkpfanne betrifft, so wird insbesondere auf die Gelenkpfanne gemäss Fig. 4 Bezug genommen, weil dieses Ausführungsbeispiel gegenüber dem Ausführungsbeispiel gemäss Fig. 1-3 die Besonderheit der zusätzlichen Finnen 25 (oder alternativ Zähne) aufweist. Insbesondere wird dabei auf die Herstellung der Aussenschale 2 eingegangen, weil der Einsatz 3 einschliesslich des zylindrischen Zapfens 34 auf konventionelle Art und Weise ohne Besonderheiten hergestellt werden kann.

Dies ist speziell wegen der Finnen 25 (bzw. alternativ Zähne) bei der Aussenschale 2 anders. Bevor nämlich die Schlitze 22 in die Aussenschale eingefräst werden können, müssen zuvor die Finnen 25 aussen auf der Aussenfläche der Aussenschale 2 hergestellt worden sein. Werden alternativ diskrete Zähne anstelle von umlaufenden Finnen auf der Aussenfläche der Aussenschale 2 gewünscht, so muss nach dem Drehen von Finnen auch noch ein Fräsvorgang in meridianer Richtung erfolgen, der die Finnen vor dem Fräsen der Schlitze 22 in diskrete Zähne unterteilt. Nach dem Fräsen der Schlitze 22 ist es nämlich aufgrund der "Weichheit" der als "Biegefedern" fungierenden T-förmigen Segmente 20 nicht mehr möglich, diese in der beschriebenen Art und Weise zu bearbeiten, ohne nicht eine plastische Verformung der Aussenschale zu riskieren bzw. zu erwirken.

## Patentansprüche

1. Künstliche Gelenkpfanne (1) mit einer Aussenschale (2) zur Befestigung im Knochen und mit einem in dieser Aussenschale (2) angeordneten Einsatz (3) zur Aufnahme eines Gelenkkopfs, bei welcher Gelenkpfanne die Aussenschale (2) mindestens ein bewegliches Segment (20) aufweist, das durch in Meridianen verlaufende Schlitze begrenzt ist, und bei welcher Gelenkpfanne der Einsatz (3) so ausgebildet ist, dass zwischen der Innenfläche der Aussenschale (2) und der Aussenfläche des Einsatzes (3) ein lichter Raum (23) gebildet wird, und bei welcher Gelenkpfanne das bewegliche Segment (20) der Aussenschale (2) zusätzlich zu den in Meridianen verlaufenden Schlitzen auch noch durch in äquatorialer Richtung verlaufende Schlitze (22) begrenzt ist.

2. Künstliche Gelenkpfanne nach Anspruch 1, bei welcher die Schlitze (22), die das bewegliche Segment (20) begrenzen, so verlaufen, dass das Segment (20) sowohl in radialer Richtung als auch in äquatorialer Richtung beweglich ist.

3. Künstliche Gelenkpfanne nach Anspruch 2, bei welcher die Schlitze (22), welche das bewegliche Segement (20) der Aussenschale (2) begrenzen, so verlaufen, dass die Aussenschale (2) mindestens ein T-förmiges Segment (20) aufweist, welches seinen Fuss (200) im Polbereich hat und sein Dach (201) im äquatorialen Bereich.

4. Künstliche Gelenkpfanne nach Anspruch 3, bei welcher die Aussenschale (2) drei T-förmige Segmente (20) aufweist.

5. Künstliche Gelenkpfanne nach einem der vorangehenden Ansprüche, dadurch gekennzeichnet, dass die Dicke der Aussenschale (29 in Richtung vom Polbereich zum äquatorialen Bereich hin abnimmt.

6. Künstliche Gelenkpfanne nach einem der vorangehenden Ansprüche, dadurch gekennzeichnet, dass die Aussenschale (2) im äquatorialen Bereich über die Fortsetzung (K) der Kontur des Aussenschale (2) nach aussen hin vorstehend ausgebildet ist, und dass in diesem Bereich der Aussenschale (2) auf der Aussenwand Mittel (25) zur Befestigung der Aussenschale (2) im Knochen vorgesehen sind.

7. Künstliche Gelenkpfanne nach einem der vorangehenden Ansprüche, bei welcher der Einsatz (3) im Bereich des Pols der Aussenschale (2) fest mit der Aussenschale (2) verbunden ist.

8. Künstliche Gelenkpfanne nach Anspruch 7, bei welcher die Verbindung des Einsatzes (3) mit der Aussenschale (2) durch einen zylindrischen Zapfen (34) gebildet wird, welcher in eine entsprechende Öffnung (24) der Aussenschale (2) eingepresst ist.

9. Künstliche Gelenkpfanne nach einem der vorangehenden Ansprüche, bei welcher sowohl die Aussenschale (2) als auch der Einsatz (3) metallisch sind, wobei die Aussenschale (2) vorzugsweise aus Titan bzw. aus einer Titanlegierung und der Einsatz (3) vorzugsweise aus einer Kobalt-Chrom-Legierung hergestellt ist.
